# EUROPEAN PATENT APPLICATION

(11) **EP 3 338 644 A1**
(43) Date of publication of application: **27.06.2018**
(21) Application number: 16792243.4
(22) Date of filing: 12.05.2016
(51) Int. Cl.: A61B 10/00, A61B 50/30

(54) **TOP FOR CUTTING SAMPLES**

(30) Priority: 13.05.2015 ES 201530558 U
(71) Applicant: Servicio Andaluz de Salud, 41071 Sevilla (ES)
(72) Inventor: VELASCO BUENO, José Manuel, 41071 Sevilla (ES)
(74) Representative: ABG Patentes, S.L.
(86) International application number: PCT/ES2016/070358
(87) International publication number: WO 2016/181010

(57) **Abstract**

The invention relates to a cap for cutting catheter specimens, integrating a cutting element particularly designed for cutting the tip of a catheter at the same time it is introduced into a bottle, making the microbiological diagnosis of possible nosocomial diseases easier as it prevents possible contaminations of the specimen as a result of mishandling. It also prevents medical personnel from accidentally cutting themselves with a scalpel or scissors.

## Description

### Object of the Invention

The present invention belongs generally to the field of medicine, and more particularly to devices used for taking specimens.

The object of the present invention is a cap for cutting catheter specimens, integrating a cutting element particularly designed for cutting the tip of a catheter at the same time it is introduced into a bottle.

### Background of the Invention

The use of intravascular catheters for diagnostic or therapeutic purposes is quite common, particularly in patients who are in critical condition or who have serious acute or chronic pathologies. However, a drawback associated with their use relates to the onset of infections, which constitute the main cause of nosocomial bacteremia and are related with high morbidity and mortality.

In the event of a suspected catheter-related infection, the catheter must first be removed. Then, for the purpose of helping clinical personnel to determine if the patient's clinical features are in fact due to the catheter and to act accordingly, it is common to perform a culture of the tip of the catheter for microbiological analysis. The specimen to be processed is the distal segment of the intravascular catheter (usually having a length of about 3 to 5 cm). This segment must be sent to the microbiology laboratory in a sterile, preferably wide-mouth bottle.

There is no device today that is particularly designed for carrying out this task, such that the process used is essentially the following. The catheter is held above the bottle inside which the specimen is to be stored, and then the catheter is cut with the aid of a cutting element such as a scalpel or scissors. Once the tip of the catheter has been separated, it is left to fall into the bottle, which is then closed with the aid of what is usually a screw-on cap.

This method has various drawbacks, some of which are the following:
- The specimen may be contaminated as a result of the handling that is necessary for cutting the tip of the catheter.
- The medical personnel may accidentally cut themselves when using the scalpel or scissors for cutting the tip of the catheter.
- Blood and/or fluid residues present inside the catheter may splash out while cutting said catheter.

### Description of the Invention

The present invention describes a device that is particularly designed for cutting specimens for introducing them into a bottle in a simple and completely safe manner. The device is essentially formed by a cap provided with means that allow cutting the specimen while at the same time closing the bottle which will house said specimen.

In this document, the term "bottle" refers to a housing, such as a receptacle, container, packaging, etc., regardless of its shape or material, that is capable of housing therein a specimen such as a catheter tip of between 3 and 5 cm or similar.

In the present document, the term "cap" refers to any element that is particularly designed for closing the bottle for the purpose of preventing both the specimen housed therein from coming out and for preventing contamination from getting in. The coupling mechanism between the cap and the bottle can be of any type, such as for example a screw-on or press-fit coupling, provided that the bottle is closed in a secure manner to be sent to a laboratory. Evidently, both the bottle and the cap must be sterile.

In the present document, the term "specimen" must be interpreted in a broad manner covering not only catheter tips, but also any other type of specimen in general that is susceptible to being cut using the cap of the invention. These specimens can be both organic and inorganic, for example, intubation tubes, drainage tubes, etc.

The invention relates to a cap configured for being coupled to a bottle capable of housing the specimen to be cut, and wherein the cap comprises a longitudinal opening and cutting means configured for cutting the specimen when it is introduced through said longitudinal opening. The cutting means preferably comprise a blade movable from a first open position, in which said blade is outside the longitudinal opening, to a second closed position, in which said blade closes the longitudinal opening. The longitudinal opening is a usually circular opening made in the main plane of the cap which is perpendicular to the longitudinal axis of the cap and the bottle. Therefore, the actual passage of the blade through said longitudinal opening from one position to another causes the sectioning of the specimen, which falls into the bottle.

The user therefore only has to place the cap on the bottle, introduce a piece of the specimen to be cut through the opening of the cap, and operate the cutting means. The piece of the specimen is sectioned and falls directly into the bottle. This device completely prevents the risk of contamination of the specimen, as well as preventing cuts or fluids that may possibly be present in the specimen from splashing out. It also greatly minimizes the risk of the user being cut or pricked during the process. Furthermore, it allows the process to be carried out by a single person in a quick, clean, and simple manner.

The cutting means can be implemented in different ways. For example, the blade could move from the first open position to the second closed position automatically when the user couples the cap to the bottle. To that end, the blade could be attached to operating means cooperating with the edge or the walls of the bottle itself.

However, in a preferred embodiment of the invention the movement of the blade from the first open position to the second closed position is carried out by hand. More preferably, the cap comprises a transverse opening provided with a transverse track along which the blade is movable from the first open position to the second closed position. In this context, the "transverse" opening refers to an opening perpendicular to the longitudinal direction described above. It is, therefore, an opening made in the side wall of the cap.

In another preferred embodiment, the transverse track comprises first locking means cooperating with first complementary locking means of the blade for locking said blade in the second closed position. More preferably, the first locking means of the transverse track are first notches configured for being coupled, when the blade is in the second closed position, to first flanges constituting the first complementary locking means of the blade. The blade is thereby locked in its closed position after the specimen has been cut, preventing contamination from getting in through the longitudinal opening.

In yet another preferred embodiment, the transverse track comprises second locking means cooperating with second complementary locking means of the blade for preventing said blade from being taken out of the transverse opening. More preferably, the second locking means of the transverse track are second notches configured for being coupled, when the blade is in the first open position, to second flanges constituting the second complementary locking means of the blade. The possibility of a user inadvertently taking the blade out of its track is thereby prevented, with the possibility of accidental cutting or pricking being limited as much as possible.

In still yet another preferred embodiment, the blade comprises a blunt base projecting through the transverse opening when the blade is in the first open position for driving said blade to the second closed position by hand. The user then simply has to push the base of the blade which initially projects transversely from the cap.

In yet another preferred embodiment, the blade comprises a raised portion located in a position coinciding with the longitudinal opening when it is in the second closed position for maximizing tightness of the closure of said longitudinal opening.

The invention also relates to an assembly of parts comprising a cap like the one described above, and a bottle that can be coupled to said cap. This sterile bottle-cap assembly can be sold as a single article intended for taking specimens.

### Brief Description of the Drawings

Figures 1a and 1b show a perspective view of a bottle to which there is coupled a cap according to the invention with the blade in the first open position and in the second closed position, respectively.
Figures 2a and 2b show a longitudinal section parallel to the direction of movement of the blade of a cap according to the invention with the blade in the first open position and in the second closed position, respectively.
Figure 3 shows a perspective view of the cap of the invention viewed from the lower side.
Figure 4 shows a perspective view of a longitudinal section perpendicular to the direction of movement of the blade of a cap according to the invention.
Figure 5 shows a bottom view of the cap of the invention in which the position of the notches and flanges can be seen.

### Preferred Embodiment of the Invention

An example of a cap (1) according to the invention is described below according to the attached drawings. In this example, reference will be made to the cutting of a specimen consisting of the tip of a catheter, although it is understood that the cap (1) of the invention can be used for cutting other types of specimens.

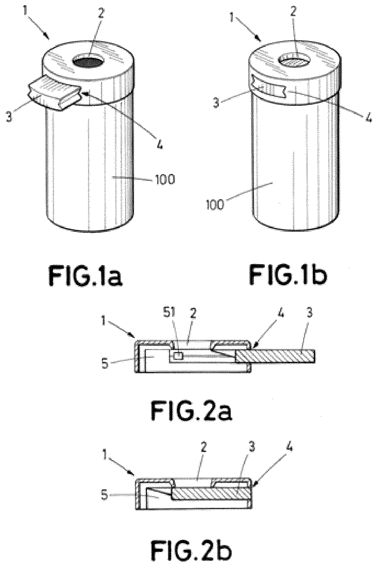

Figures 1a and 1b show respective perspective views of a bottle (100) to which there is coupled the cap (1) and of the cap (1) itself according to the present invention. Both the cap (1) and the bottle (100) are sterile, are cylindrical in shape, and are coupled to one another by means of a thread (not shown). The cap (1) is formed by a planar circular portion which closes the open end of the bottle (100) and a cylindrical side portion on the inner surface of which the thread for the fixing thereof to said bottle (100) is located. The cap (1) furthermore has a circular longitudinal opening (2) located in the center of the planar circular portion and an essentially rectangular transverse opening (4) located on the cylindrical side portion thereof. Furthermore, as can be best seen in later figures, the blade (3) is formed by a blunt base at the proximal end projecting through the transverse opening (4) to be operated by the user, and a cutting element located at the distal end for sectioning the tip of the catheter.

In Figure 1a, the blade (3) is in the first open position, such that it does not cover the longitudinal opening (2) of the cap (1) through which the tip of the catheter will be introduced for separating the specimen. The blunt base constituting the proximal end of the blade (3) projects through the transverse opening (4) so that the user can operate it. In Figure 1b, the blade (3) has already been moved until it has been completely introduced into the transverse opening (4). The longitudinal opening (2) is covered by the cutting element of the blade (3) itself, whereas the blunt base is level with the outer surface of the cylindrical side portion of the cap (1). The actual movement of the blade (3) from one position to another causes the cutting of the catheter.

Figures 2a and 2b again show the blade (3) in the first open position and in the second closed position, respectively, but this time by means of a longitudinal section along a plane parallel to the direction of movement of the blade (3). It can be seen how the blade (3) moves in a rectilinear manner parallel to the planar circular portion of the cap (1), which will allow cutting the catheter in a simple and effortless manner. Furthermore, although it cannot be seen in these figures, the blade (3) can have a protuberance or raised portion designed for fitting in the circular longitudinal opening (2), and therefore be circular-shaped in this case, when the blade (3) reaches the second closed position. Tightness of the closure of the opening (2) is thereby maximized.

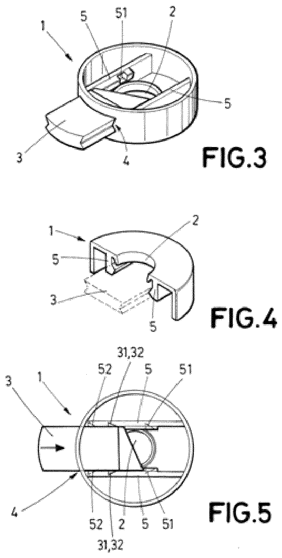

Figure 3 shows a perspective view of the cap (1) located in the inverted position during an operation for moving the blade (3) from the first open position to the second closed position. Though not depicted in these drawings, it would be possible to configure the track (5) such that the blade is covered completely by the lower face of the cap (1), obviously except for the area where the circular longitudinal opening (2) is located.

Figure 4 shows a longitudinal section along a plane perpendicular to the direction of movement of the blade (3) where the transverse track (5) along which the blade (3) slides can be seen. To that end, it can be seen how the proximal portion of the blade (3), which comprises a blunt base made of a plastic material in this example, has longitudinal grooves located on both sides and complementary with the two rails constituting the track (5). The blade (3) therefore moves at all times along the track (5) in a rectilinear manner.

As can be observed in Figure 5, as well as in Figure 2a and Figure 3, the cap (1) of the invention furthermore has first locking means to prevent the blade (3) from being able to go from the second closed position back to the first open position, and therefore preventing the opening (2) from being able to be open again once the second closed position has been reached. These first locking means comprise notches (51) made in the distal portion of the track (5) (i.e., at the opposite end of the track (5) in relation to where the transverse opening (4) is located) in conjunction with side flanges (31) located in the distal portion of the blade (3). As can be seen in the drawings, the flanges (31) are configured in a diagonal arrangement in relation to the direction of movement of the blade (3), such that once they enter the notches (51) when the blade (3) reaches the second closed position, it is no longer possible to move the blade (3) back again to the first open position.

Furthermore, the cap (1) of this example has second locking means to prevent the blade (3) from being completely taken out of and separated from the cap (1). These locking means comprise notches (52) made in the proximal portion of the track (5) (i.e., at the end of the track (5) closest to the transverse opening (4)) in conjunction with side flanges (32) of the blade (3). In this example, the side flanges (32) of the blade (3) coincide with side flanges (31), although this is not indispensable for the operation thereof. In the first open position, the side flanges (32) of the blade (3) are in the notches (52), thereby preventing being able to completely take the blade (3) out. The configuration of the flanges (32), which is identical to that described above in relation to flanges (31), allows them to come out of the notches (52) when the user pushes the blade (3) to the second closed position.

This cap (1) can be provided already screwed onto the corresponding bottle (100) with the blade (3) located in the first open position and with the side flanges (32) introduced in the notches (52), such that it prevents the blade (3) from being taken out completely. To use this article, the user must simply introduce the tip of the catheter through the longitudinal opening (2) and push the blade (3) from said first open position to the second closed position. The blade (3) slides along the track (5), first sectioning the catheter and then closing the longitudinal opening (2). This entire process can be carried out using only two hands, and it can therefore be carried out by a single person. Since the cap (1) is already screwed onto the bottle (100), the latter is closed once the cutting has been performed, and the assembly can be sent to the laboratory for analysis.

## Claims

1. A cap (1) for cutting specimens, where the cap (1) is configured for being coupled to a bottle (100) capable of housing the specimen, **characterized in that** it comprises a longitudinal opening (2) and cutting means (3) configured for cutting the specimen when said specimen is introduced through said longitudinal opening (2).

2. The cap (1) according to claim 1, wherein the cutting means (3) comprise a blade (3) movable from a first open position, in which said blade (3) is outside the longitudinal opening (2), to a second closed position, in which said blade (3) closes the longitudinal opening (2).

3. The cap (1) according to claim 2, wherein the movement of the blade (3) from the first open position to the second closed position is carried out by hand.

4. The cap (1) according to claim 3, comprising a transverse opening (4) provided with a transverse track (5) along which the blade (3) is movable from the first open position to the second closed position.

5. The cap (1) according to claim 4, wherein the transverse track (5) comprises first locking means cooperating with first complementary locking means of the blade (3) for locking said blade (3) in the second closed position.

6. The cap (1) according to claim 5, wherein the first locking means of the transverse track (5) are first notches (51) configured for being coupled, when the blade (3) is in the second closed position, to first flanges (31) constituting the first complementary locking means of the blade (31).

7. The cap (1) according to any of claims 4 to 6, wherein the transverse track (5) comprises second locking means cooperating with second complementary locking means of the blade (3) for preventing said blade (3) from being taken out of the transverse opening (4).

8. The cap (1) according to claim 7, wherein the second locking means of the transverse track (5) are second notches (52) configured for being coupled, when the blade (3) is in the first open position, to second flanges (32) constituting the second complementary locking means of the blade (3).

9. The cap (1) according to any of claims 4 to 8, wherein the blade (3) comprises a blunt base projecting through the transverse opening (4) when the blade (3) is in the first open position such that it allows driving said blade (3) to the second closed position by hand.

10. The cap (1) according to claim 9, wherein the length of the blade (3) is such that its blunt base is flush with the side surface of the cap (1) when said blade (3) is in the second closed position.

11. The cap (1) according to any of the preceding claims, wherein the blade (3) comprises a raised portion located in a position coinciding with the longitudinal opening (2) when it is in the second closed position for maximizing tightness of the closure of said longitudinal opening (2).

12. An assembly of parts, **characterized in that** it comprises:
- a cap (1) according to any of preceding claims 1 to 11; and
- a bottle (100) that can be coupled to said cap (1).
